# EUROPÄISCHE PATENTANMELDUNG

(11) **EP 0 823 242 A2**
(43) Veröffentlichungstag der Anmeldung: **11.02.1998**
(21) Anmeldenummer: 97110902.0
(22) Anmeldetag: 02.07.1997
(51) Int. Cl.: A61B 17/56, A61B 17/16

(54) **Instrument zur Bearbeitung des Markraumes**

(30) Priorität: 08.08.1996 DE 19631984
(71) Anmelder: Aesculap AG & Co. KG, 78532 Tuttlingen (DE)
(72) Erfinder: Hermle, Thomas, D-78628 Rottweil (DE)
(74) Vertreter: Böhme, Ulrich Hoeger, Stellrecht & Partner

(57) **Zusammenfassung**

Um bei einem chirurgischen Implantationsinstrument zur Bearbeitung des Markraumes eines Röhrenknochens mit einem Handgriff und mindestens einem mit diesem Griff verbindbaren Werkzeug eine einfache Entnahme des Werkzeuges auch dann zu ermöglichen, wenn es unbeabsichtigt vom Instrument getrennt wird, wird vorgeschlagen, daß der Handgriff mit einer länglichen Aufnahmehülse versehen ist, in die ein länglicher Schaft des Werkzeuges einführbar ist, daß Verriegelungsmittel zur axialen und zur drehfesten Festlegung des Schaftes in der Aufnahmehülse vorgesehen sind und daß der Schaft des Werkzeuges in seiner verriegelten Stellung über einen wesentlichen Teil der Länge der Aufnahmehülse in diese eintaucht.

## Beschreibung

Die Erfindung betrifft ein chirurgisches Implantationsinstrument zur Bearbeitung des Markraumes eines Röhrenknochens mit einem Handgriff und mindestens einem mit diesem drehfest verbindbaren Werkzeug.

Bei der Implantation von Endoprothesen in den Markraum von Röhrenknochen, beispielsweise bei dem Einsetzen einer Hüftgelenksendoprothese, ist es notwendig, den Markraum für die Aufnahme des Schaftes der Endoprothese vorzubereiten. Diese Bearbeitungsvorgänge müssen in der Tiefe des Markraumes ausgeführt werden, dabei sind die unterschiedlichsten Bearbeitungsvorgänge notwendig, beispielsweise Ausmessen, Aufweiten, Feilen, Gewebeentnahme etc. Für all diese Vorgänge werden unterschiedliche Werkzeuge benötigt, die handgeführt durch entsprechende Bewegungen im Markraum Bearbeitungsschritte vornehmen sollen, beispielsweise werden diese Werkzeuge in Längsrichtung hin- und hergeschoben oder um die Längsachse des Markraumes verdreht.

Es ist bekannt, zu diesem Zweck einen Handgriff mit einem entsprechenden Werkzeug zu verbinden, der Handgriff bleibt außerhalb des Markraumes, das Werkzeug taucht zu dessen Bearbeitung in den Markraum ein.

Dabei besteht die Gefahr, daß bei einer unbeabsichtigten Trennung des Werkzeuges vom Handgriff das Werkzeug im Inneren des Markraumes verbleibt und nur unter großten Schwierigkeiten aus dem Markraum entfernt werden kann.

Es ist Aufgabe der Erfindung, ein solches Implantationsinstrument so auszubilden, daß bei einer unbeabsichtigten Trennung des Werkzeuges vom Handgriff das Werkzeug ohne Schwierigkeiten aus dem Markraum entnommen werden kann.

Diese Aufgabe wird bei einem chirurgischen Implantationsinstrument der eingangs beschriebenen Art erfindungsgemäß dadurch gelöst, daß der Handgriff mit einer länglichen Aufnahmehülse versehen ist, in die ein länglicher Schaft des Werkzeuges einführbar ist, daß Verriegelungsmittel zur axialen und zur drehfesten Festlegung des Schaftes in der Aufnahmehülse vorgesehen sind und daß der Schaft des Werkzeuges in seiner verriegelten Stellung über einen wesentlichen Teil der Länge der Aufnahmehülse in diese eintaucht.

Die längliche Aufnahmehülse dieses Instrumentes stellt die Verbindung zwischen dem Handgriff her, der außerhalb des Markraumes eingesetzt wird, und dem Werkzeug, das in der Tiefe des Markraumes zum Einsatz kommt.

Durch die Ausbildung der Verbindung zwischen Handgriff und Markraum durch das Eintauchen eines länglichen Schaftes in die Aufnahmehülse wird erreicht, daß bei einer unbeabsichtigten Trennung das Werkzeug mit dem länglichen Schaft aus dem Markraum hervorsteht, so daß der Chirurg das Werkzeug an dem vorstehenden Schaft ohne weiteres ergreifen und aus dem Markraum herausziehen kann.

Dabei ist es günstig, wenn die Eintauchtiefe des Schaftes in der verriegelten Stellung größer ist als die halbe Länge der Aufnahmehülse.

Einen weiteren Vorteil erhält man durch diese Ausgestaltung dadurch, daß der Schaft und die Aufnahmehülse über eine große Länge aneinander anliegen, so daß die Verbindung auch aus diesem Grunde eine hohe Festigkeit gegen Biegung erhält.

Vorzugsweise liegt die Länge des in die Aufnahmehülse eintauchenden Schaftes zwischen 10 cm und 25 cm.

Bei einer bevorzugten Ausführungsform ist vorgesehen, daß der Schaft in der Aufnahmehülse durch eine Bajonettverriegelung festlegbar ist.

Dazu kann der Schaft einen seitlichen Vorsprung tragen, der in einen in der Wand der Aufnahmehülse von deren Sternkante zunächst axial und dann in Umfangsrichtung verlaufenden Schlitz eintaucht.

Günstig ist es, wenn der Schlitz an seinem der Stirnkante abgewandten Ende eine in Richtung auf die Stirnkante verlaufende Erweiterung trägt. In diese Erweiterung tritt der Vorsprung des Schaftes in der verriegelten Stellung ein, so daß ein unbeabsichtigtes Lösen der Bajonettverbindung vermieden wird.

Gemäß einer bevorzugten Ausführungsform kann weiterhin vorgesehen sein, daß im Innern der Aufnahmehülse ein in dessen Längsrichtung verschieblich gelagerter und in Richtung auf deren Stirnkante durch eine Feder verschobener Stößel angeordnet ist. Dieser Stößel versucht, den eingesetzten Schaft aus der Aufnahmehülse herauszuschieben, dabei tritt die Verlängerung des Schaftes in die Erweiterung am Ende des Bajonettschlitzes ein, so daß ein Lösen der Bajonettverbindung nur gegen die Kraft der den Stößel verschiebenden Feder möglich ist.

Der Stößel kann mit einem durch eine seitliche Öffnung in der Aufnahmehülse hindurchtretenden Griffelement verbunden sein, mit dessen Hilfe er entgegen der Feder verschoben werden kann, so daß dadurch das Lösen der Bajonettverbindung ermöglicht wird.

Insbesondere kann das Griffelement quer in den Stößel eingeschraubt sein.

Es ist weiterhin vorteilhaft, wenn der Stößel in der Aufnahmehülse gegen eine Drehung um seine Längsachse gesichert ist und bei Anlage an einem in die Aufnahmehülse eingesetzten Schaft eine formschlüssige Verbindung mit diesem eingeht. Der Stößel nimmt somit die wesentlichen Drehkräfte des Werkzeuges auf und leitet diese in den Handgriff ein.

Beispielsweise kann die formschlüssige Verbindung eine Nut-Feder-Verbindung sein, es sind aber auch alle anderen Verbindungen denkbar, die beispielsweise zwischen Schraubendreher und Schrauben bekannt sind, beispielsweise eine Kreuzschlitz-Verbindung oder eine Imbus-Verbindung.

Bei einer bevorzugten Ausführungsform der Erfindung ist vorgesehen, daß auf der Aufnahmehülse ein Ring verschiebbar gelagert ist, der in verschiedenen Positionen auf der Aufnahmehülse festlegbar ist. Dieser Ring wirkt als Markierungsring und kann dem Chirurg die Eintauchtiefe des Werkzeuges in den Markraum anzeigen, d. h. den Abstand des Werkzeuges von dem in einer bestimmten Position festgelegten Ring.

Der Ring kann beispielsweise auf der Aufnahmehülse reibend gehalten sein, so daß er unter Überwindung dieser Reibungskraft in seiner Position verstellbar ist.

Grundsätzlich kann das aus Handgriff und Aufnahmehülse bestehende Instrumententeil mit einem bestimmten Werkzeug kombiniert werden. Es ist jedoch besonders vorteilhaft, wenn einem Handgriff mit Aufnahmehülse ein Satz unterschiedlicher Werkzeuge mit in gleicher Weise in die Aufnahmehülse einsetzbarem Schaft zugeordnet ist. Dasselbe aus Handgriff und Aufnahmehülse bestehende Instrumententeil kann dann in der gewünschten Weise mit unterschiedlichen Werkzeugen kombiniert werden, wobei diese in einfacher Weise axial und in Drehrichtung festgelegt in die Aufnahmehülse eingesetzt werden können. Falls diese Werkzeuge unterschiedliche Längsabmessungen haben, kann dies durch Verschiebung des Ringes auf der Aufnahmehülse berücksichtigt werden.

Die nachfolgende Beschreibung einer bevorzugten Ausführungsform der Erfindung dient im Zusammenhang mit der Zeichnung der näheren Erläuterung. Es zeigen:

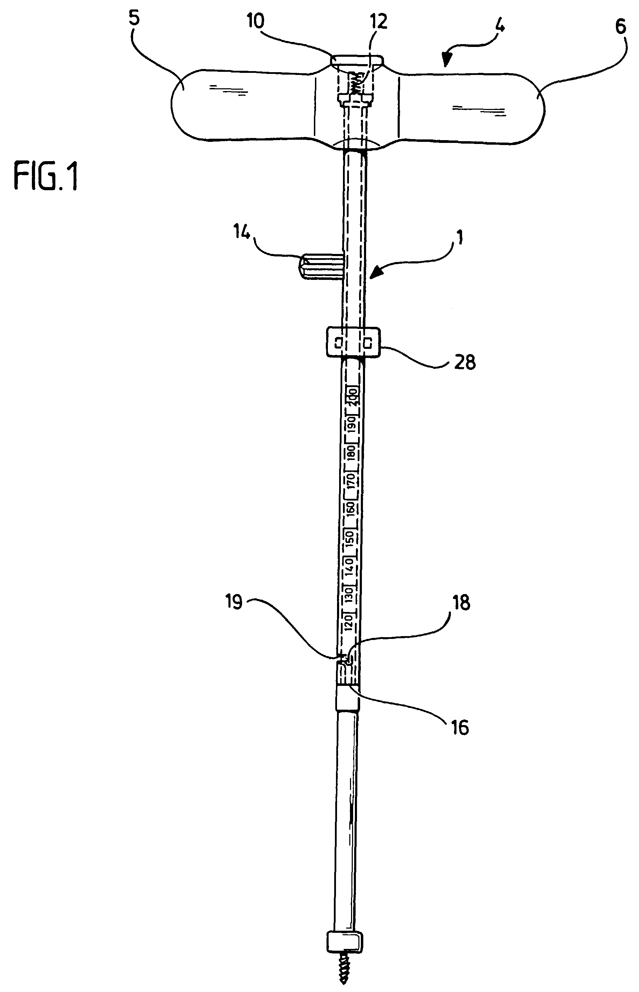

- Figur 1:: eine Seitenansicht eines Implantationsinstruments mit eingesetztem Werkzeug;

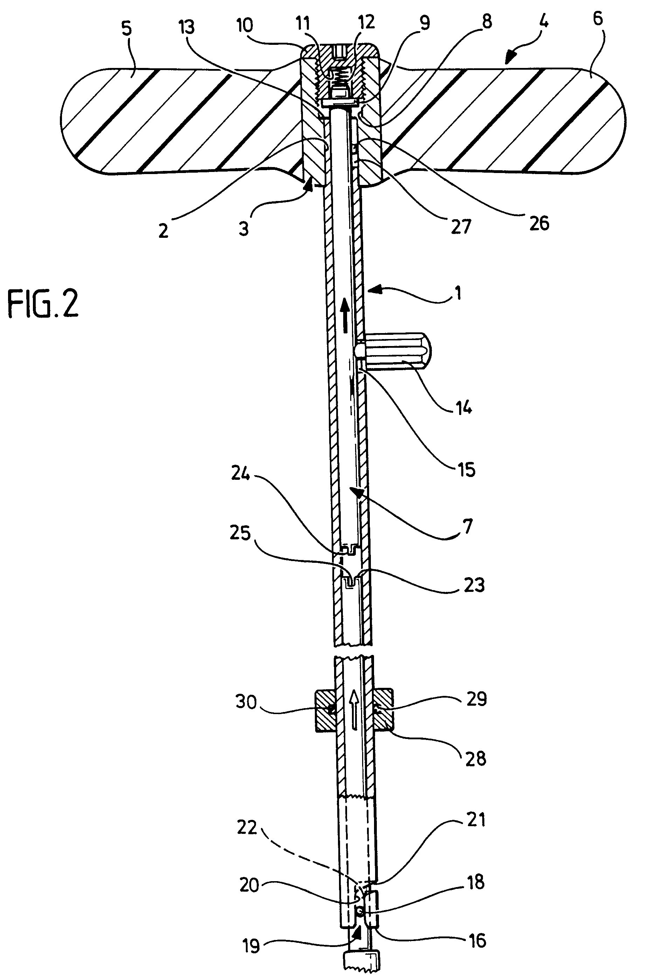

- Figur 2:: eine Längsschnittansicht des Aufnahmeteils des Instruments der Figur 1 beim Einsetzen eines Werkzeuges und

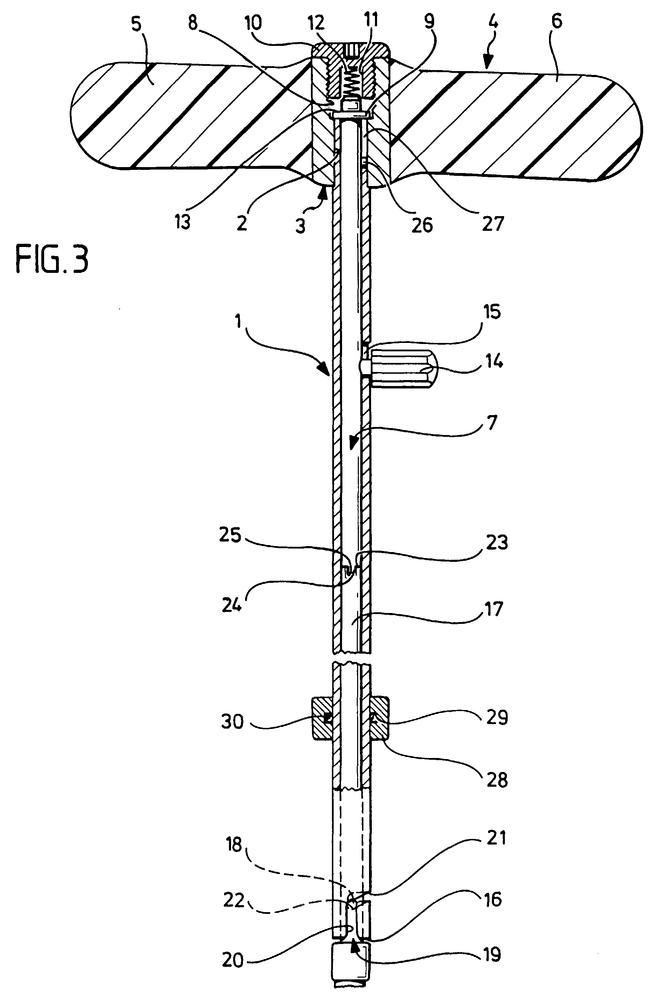

- Figur 3:: eine Ansicht ähnlich Figur 2 mit eingesetztem Werkzeug.

Das in der Zeichnung dargestellte Implantationsinstrument umfaßt eine rohrförmige Aufnahmehülse 1, die mit ihrem oberen Ende in eine sich stufenförmig erweiternde Bohrung 2 einer Haltebuchse 3 eingesetzt und mit dieser dauerhaft verbunden ist. Die Haltebuchse 3 bildet den zentralen Teil eines Handgriffs 4 mit zwei quer von der Haltebuchse 3 abstehenden, einander diametral gegenüberliegenden Griffbereichen 5 und 6. Der Handgriff 4 und die Aufnahmehülse 1 sind auf diese Weise in axialer Richtung und in Drehrichtung starr miteinander verbunden.

Im Innern der Aufnahmehülse 1 ist ein Stößel 7 längsverschieblich gelagert, der mit seinem oberen Ende in den erweiterten Bereich 8 der Bohrung 2 eintritt und dort einen Kragen 9 trägt, der die Einschubtiefe des Stößels 7 in die Aufnahmehülse 1 begrenzt. In den erweiterten Bereich 8 der Bohrung 2 ist ein Verschlußstopfen 10 eingeschraubt, der in einer Sacklochbohrung 11 eine Schraubenfeder 12 aufnimmt, die sich einerseits am Boden der Sacklochbohrung 11 und andererseits an der Stirnseite des Stößels 7 abstützt und diesen dadurch in die Aufnahmehülse 1 einschiebt, bis der Kragen 9 an der oberen Stirnkante 13 der Aufnahmehülse 1 anliegt. Gegen die Wirkung der Schraubenfeder 12 kann der Stößel 7 im Innern der Aufnahmehülse 1 um einige Millimeter verschoben werden. Diese Verschiebung wird möglich durch ein seitliches Griffelement 14, welches durch eine seitliche Langlochöffnung 15 in der Aufnahmehülse 1 hindurchtritt und dort seitlich in den Stößel 7 eingeschraubt ist.

Der Stößel 7 erstreckt sich nur soweit in die Aufnahmehülse hinein, daß ein wesentlicher Teil der Aufnahmehülse 1 von deren unterer Stirnkante 16 her freibleibt.

Von dieser unteren Stirnkante 16 her wird in die Aufnahmehülse 1 ein länglicher Schaft 17 eines Werkzeuges 18 eingeschoben. Dieser Schaft 17 trägt einen seitlichen Stift 18, der in einen Schlitz 19 in der Wand der Aufnahmehülse 1 eintritt. Der Schlitz 19 beginnt an der unteren Stirnkante 16 der Aufnahmehülse 1 und verläuft zunächst in einem ersten Abschnitt 20 parallel zur Längsachse der Aufnahmehülse 1, in einem daran anschließenden Abschnitt 21 in Umfangsrichtung und endet in einer wieder in Längsrichtung der Aufnahmehülse 1 in Richtung auf die vordere Stirnkante 16 gerichteten Erweiterung 22.

Der Schaft 17 ist so lang, daß er beim Einsetzen mit seiner Stirnseite 23 am Stößel 7 zur Anlage kommt. In diesem Kontaktbereich ergibt sich bei der Anlage ein Formschluß dadurch, daß der Stößel 7 eine diametral verlaufende Feder 24 trägt, die in eine komplementäre Nut 25 in der Stirnseite 23 des Schaftes 17 eingreift.

Beim Einsetzen des Schaftes wird dieser von der Stirnkante 16 der Aufnahmehülse 1 her in diese eingeschoben, wobei der Stift 18 in den Abschnitt 20 des Schlitzes 19 eintritt. Außerdem tritt die Feder 24 des Stößels 7 in die Nut 25 des Schaftes 17 ein, so daß beim weiteren Einschieben des Schaftes auch der Stößel 7 entgegen der Wirkung der Schraubenfeder 12 verschoben wird. Der Stift 18 gelangt bei dieser Einschubbewegung, die von einer Drehung des Schaftes 17 begleitet wird, schließlich in die Erweiterung 22 des Schlitzes 19 und wird unter der Wirkung des federbelasteten Stößels 7 in diese Erweiterung 22 hineingedrückt. Dadurch ist eine Bajonettverriegelung des Schaftes 17 erreicht, außerdem eine drehfeste Verbindung des Schaftes 17 mit dem Stößel 7.

Dieser Stößel 7 ist in der Haltebuchse 3 zwar längsverschieblich, jedoch drehfest gelagert. Diese drehfeste Lagerung wird erreicht durch einen radial abstehenden Stift 26, der in einer axialen Nut 27 geführt ist, durch einen achsparallelen Schlitz im oberen Teil der Aufnahmehülse 1 gebildet wird.

Zur Lösung der beschriebenen Verbindung genügt es, den Stößel 7 mit Hilfe des Griffelementes 14 entgegen der Schraubenfeder 12 zu verschieben, durch eine Drehbewegung und durch ein axiales Herausziehen des Schaftes 17 kann das Werkzeug dann wieder aus der Aufnahmehülse 1 entnommen werden.

Auf diese Weise können in dieselbe Aufnahmehülse 1 verschiedene Werkzeuge eingesetzt werden, die alle in gleicher Weise einen länglichen Schaft 17 aufweisen.

Auf der Aufnahmehülse 1 ist ein Ring 28 in Längsrichtung verschiebbar gelagert. In einer Umfangsnut 29 an der Innenwand des Ringes 28 ist ein O-Ring 30 aufgenommen, der zwischen dem Ring 28 und der Außenwand der Aufnahmehülse 1 eine Reibkraft erzeugt, durch die der Ring 28 in einer einmal eingenommenen Stellung verbleibt. Die Reibungskraft ist jedoch nur so groß, daß eine Verschiebung des Ringes 28 möglich ist. Der Chirurg kann daher diesen Ring 28 in unterschiedlicher Längsposition auf der Aufnahmehülse 1 anordnen und so eine Markierung für den Abstand zum Werkzeug festlegen, so daß er auch bei in einem in den Markraum eingesetzten Werkzeug abschätzen kann, in welcher Tiefe des Markraumes sich das Werkzeug befindet.

Der Schaft 17 ist so lang ausgebildet, daß er auch dann noch aus dem Markraum hervorsteht, wenn das Werkzeug tief in den Markraum eintaucht und wenn die Verbindung zwischen dem Werkzeug und der Aufnahmehülse gelöst sein sollte. Dies erleichtert die Entnahme des Werkzeuges bei unbeabsichtigter Trennung, es ermöglicht aber auch, gegebenenfalls ein in den Markraum eingesetztes Werkzeug mit einer anderen Aufnahmehülse zu verbinden. Dies könnte wünschenswert sein, wenn für einen bestimmten Bearbeitungsgang aus ergonomischen Gründen ein anderer Handgriff 4 gewünscht wird oder eine Aufnahmehülse mit einer unterschiedlichen Länge.

## Patentansprüche

1. Chirurgisches Implantationsinstrument zur Bearbeitung des Markraumes eines Röhrenknochens mit einem Handgriff und mindestens einem mit diesem drehfest verbindbaren Werkzeug, dadurch gekennzeichnet, daß der Handgriff (4) mit einer länglichen Aufnahmehülse (1) versehen ist, in die ein länglicher Schaft (17) des Werkzeuges einführbar ist, daß Verriegelungsmittel (18, 19; 25, 26) zur axialen und zur drehfesten Festlegung des Schaftes (17) in der Aufnahmehülse (1) vorgesehen sind und daß der Schaft (17) des Werkzeuges in seiner verriegelten Stellung über einen wesentlichen Teil der Länge der Aufnahmehülse (1) in diese eintaucht.

2. Instrument nach Anspruch 1, dadurch gekennzeichnet, daß die Eintauchtiefe des Schaftes (17) in der verriegelten Stellung größer ist als die halbe Länge der Aufnahmehülse (1).

3. Instrument nach Anspruch 1 oder 2, dadurch gekennzeichnet, daß die Länge des in die Aufnahmehülse (1) eintauchenden Schaftes (17) zwischen 10 cm und 25 cm liegt.

4. Instrument nach einem der voranstehenden Ansprüche, dadurch gekennzeichnet, daß der Schaft (17) in der Aufnahmehülse (1) durch eine Bajonettverriegelung (18, 19) festlegbar ist.

5. Instrument nach Anspruch 4, dadurch gekennzeichnet, daß der Schaft (17) einen seitlichen Vorsprung (18) trägt, der in einen in der Wand der Aufnahmehülse (1) von deren Stirnkante (16) zunächst axial und dann in Umfangsrichtung verlaufenden Schlitz (19) eingreift.

6. Instrument nach Anspruch 5, dadurch gekennzeichnet, daß der Schlitz (19) an seinem der Stirnkante (16) abgewandten Ende einen in Richtung auf die Stirnkante (16) verlaufende Erweiterung (22) trägt.

7. Instrument nach einem der voranstehenden Ansprüche, dadurch gekennzeichnet, daß im Inneren der Aufnahmehülse (1) ein in dessen Längsrichtung verschieblich gelagerter und in Richtung auf deren Stirnkante (16) durch eine Feder (12) verschobener Stößel (7) angeordnet ist.

8. Instrument nach Anspruch 7, dadurch gekennzeichnet, daß der Stößel (7) mit einem durch eine seitliche Öffnung (15) in der Aufnahmehülse (1) hindurchtretenden Griffelement (14) verbunden ist.

9. Instrument nach Anspruch 8, dadurch gekennzeichnet, daß das Griffelement (14) quer in den Stößel (7) eingeschraubt ist.

10. Instrument nach einem der Ansprüche 7 bis 9, dadurch gekennzeichnet, daß der Stößel (7) in der Aufnahmehülse (1) gegen eine Drehung um seine Längsachse gesichert ist und bei Anlage an einem in die Aufnahmehülse (1) eingesetzten Schaft (17) eine formschlüssige Verbindung (24, 25) mit diesem eingeht.

11. Instrument nach Anspruch 10, dadurch gekennzeichnet, daß die formschlüssige Verbindung (24, 25) eine Nut-Feder-Verbindung ist.

12. Instrument nach einem der voranstehenden Ansprüche, dadurch gekennzeichnet, daß auf der Aufnahmehülse (1) ein Ring (28) verschiebbar gelagert ist, der in verschiedenen Positionen auf der Aufnahmehülse (1) festlegbar ist.

13. Instrument nach Anspruch 12, dadurch gekennzeichnet, daß der Ring (28) auf der Aufnahmehülse (1) reibend gehalten ist.

14. Instrument nach einem der voranstehenden Ansprüche, dadurch gekennzeichnet, daß einem Handgriff (4) mit Aufnahmehülse (1) ein Satz unterschiedlicher Werkzeuge mit in gleicher Weise in die Aufnahmehülse (1) einsetzbarem Schaft (17) zugeordnet ist.
